# EUROPÄISCHE PATENTSCHRIFT

(11) **EP 0 833 943 B1**
(45) Veröffentlichungstag und Bekanntmachung des Hinweises auf die Patenterteilung: **06.08.2003**
(21) Anmeldenummer: 96918687.3
(22) Anmeldetag: 12.06.1996
(51) Int. Cl.: C12Q 1/68, C12N 15/31

(54) **VERFAHREN ZUR IDENTIFIZIERUNG SEKRETORISCHER GENE AUS HELICOBACTER PYLORI**
METHOD FOR IDENTIFYING SECRETORY GENES IN HELICOBACTER PYLORI
PROCEDE POUR L'IDENTIFICATION DE GENES SECRETOIRES PROVENANT DE L'HELICOBACTER PYLORI

(30) Priorität: 12.06.1995 DE 19521312
(43) Veröffentlichungstag der Anmeldung: 08.04.1998
(73) Patentinhaber: Max-Planck-Gesellschaft zur Förderung der Wissenschaften e.V. Berlin, 80539 München (DE)
(72) Erfinder: HAAS, Rainer, Prof, Dr., D-81547 München (DE); ODENBREIT, Stefan Dr., D-81825 München (DE); MEYER, Thomas, D-72076 Tübingen (DE)
(74) Vertreter: Weiss, Wolfgang, Dipl.-Chem. Dr.
(86) Internationale Anmeldenummer: EP9602544
(87) Internationale Veröffentlichungsnummer: WO96041891

(56) Entgegenhaltungen:
- FR-A- 2 693 475
- US-A- 4 914 025
- MOLECULAR MICROBIOLOGY, Bd. 6, Nr. 13, Juni 1992, Seiten 1829-39, XP000605763 ALBANO M ET AL: "PhoA gene fusions in legionella pneumophila generated in vivo using a new transposon, MudphoA"
- GENE, Bd. 111, Nr. 1, Februar 1992, Seiten 21-26, XP000605471 TADAYYON M ET AL: "TnblaM: a transposon for directly tagging bacterial genes encoding cell envelope and secreted proteins"
- GENE, Bd. 49, September 1986, Seiten 341-49, XP000606470 BROOME-SMITH J ET AL: "A vector for the construction of translational fusions to TEM beta-lactamase and analysis of protein export signals and membrane protein topology"
- GENE, Bd. 130, Nr. 1, 1993, Seiten 23-31, XP000605468 HASS R ET AL: "TnMax - a versitile mini-transposon for the analysis of cloned genes and shuttle mutagenesis"
- MOLECULAR MICROBIOLOGY, Bd. 12, Nr. 2, April 1994, Seiten 307-19, XP000605827 SCHMITT W ET AL: "Genetic analysis of the Helicobacter pylori vacuolating cytotoxin: structural similarities with the IgA protease type of exported gene"
- MOLECULAR MICROBIOLOGY, Bd. 18, Nr. 4, Januar 1995, Seiten 685-701, XP000605825 REIDL J ET AL: "Characterization of Vibro cholerae bacteriophage K139 and use of a novel mini-transposon to identify a phage encode virulence factor"
- MOLECULAR MICROBIOLOGY, Bd. 12, Nr. 5, 1994, Seiten 819-31, XP000605826 KAHRS A ET AL: "Generalized transposon shuttle mutagenesis in Neisseria gonorrhoeae: a method for isolating epithelial cell invasion-defective mutants"

### SEQUENZPROTOKOLL

(1) ALLGEMEINE ANGABEN:
   (i) ANMELDER:
      (A) NAME: Max-Planck-Gesellschaft zur Foerderung der Wissenschaften e.V. Berlin
      (B) STRASSE: Hofgartenstr. 2
      (C) ORT: Muenchen
      (E) LAND: Deutschland
      (F) POSTLEITZAHL: 80359
   (ii) BEZEICHNUNG DER ERFINDUNG: Verfahren zur Identifi zierung sekretorischer Gene aus Helicobacter pylori
   (iii) ANZAHL DER SEQUENZEN: 2
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0,
         Version #1.30 (EPA)
   (v) DATEN DER JETZIGEN ANMELDUNG:
      ANMELDENUMMER: WO PCT/EP96/02545
(2) ANGABEN ZU SEQ ID NO: 1:
   (i) SEQUENZKENNZEICHEN:
      (A) LÄNGE: 1557 Basenpaare
      (B) ART: Nucleotid
      (C) STRANGFORM: beides
      (D) TOPOLOGIE: linear
   (ii) ART DES MOLEKÜLS: Genom-DNA
   (vi) URSPRÜNGLICHE HERKUNFT:
      (A) ORGANISMUS: Helicobacter pylori
   (vii) UNMITTELBARE HERKUNFT:
      (B) CLON(E): alpA
   (ix) MERKMAL:
      (A) NAME/SCHLÜSSEL: CDS
      (B) LAGE:1..1554
   (xi) SEQUENZBESCHREIBUNG: SEQ ID NO: 1:
   ATG ATA AAA AAG AAT AGA ACG CTG TTT CTT AGT CTA GCC CTT TGC GCT        48
   Met Ile Lys Lys Asn Arg Thr Leu Phe Leu Ser Leu Ala Leu Cys Ala
     1               5                  10                  15
   AGC ATA AGT TAT GCC GAA GAT GAT GGA GGG TTT TTC ACC GTC GGT TAT 96
   Ser Ile Ser Tyr Ala Glu Asp Asp Gly Gly Phe Phe Thr Val Gly Tyr
   20 25 30
   CAG CTC GGG CAA GTC ATG CAA GAT GTC CAA AAC CCA GGC GGC GCT AAA 144
   Gln Leu Gly Gln Val Met Gln Asp Val Gln Asn Pro Gly Gly Ala Lys
   35 40 45
   AGC GAC GAA CTC GCC AGA GAG CTT AAC GCT GAT GTA ACG AAC AAC ATT 192
   Ser Asp Glu Leu Ala Arg Glu Leu Asn Ala Asp Val Thr Asn Asn Ile
   50 55 60
   TTA AAC AAC AAC ACC GGA GGC AAC ATC GCA GGG GCG TTG AGT AAC GCT 240
   Leu Asn Asn Asn Thr Gly Gly Asn Ile Ala Gly Ala Leu Ser Asn Ala
   65 70 75 80
   TTC TCC CAA TAC CTT TAT TCG CTT TTA GGG GCT TAC CCC ACA AAA CTC 288
   Phe Ser Gln Tyr Leu Tyr Ser Leu Leu Gly Ala Tyr Pro Thr Lys Leu
   85 90 95
   AAT GGT AGC GAT GTG TCT GCG AAC GCT CTT TTA AGT GGT GCG GTA GGC 336
   Asn Gly Ser Asp Val Ser Ala Asn Ala Leu Leu Ser Gly Ala Val Gly
   100 105 110
   TCT GGG ACT TGT GCG GCT GCA GGG ACG GCT GGT GGC ACT TCT CTT AAC 384
   Ser Gly Thr Cys Ala Ala Ala Gly Thr Ala Gly Gly Thr Ser Leu Asn
   115 120 125
   ACT CAA AGC ACT TGC ACC GTT GCG GGC TAT TAC TGG CTC CCT AGC TTG 432
   Thr Gln Ser Thr Cys Thr Val Ala Gly Tyr Tyr Trp Leu Pro Ser Leu
   130 135 140
   ACT GAC AGG ATT TTA AGC ACG ATC GGC AGC CAG ACT AAC TAC GGC ACG 480
   Thr Asp Arg Ile Leu Ser Thr Ile Gly Ser Gln Thr Asn Tyr Gly Thr
   145 150 155 160
   AAC ACC AAT TTC CCC AAC ATG CAA CAA CAG CTC ACC TAC TTG AAT GCG 528
   Asn Thr Asn Phe Pro Asn Met Gln Gln Gln Leu Thr Tyr Leu Asn Ala
   165 170 175
   GGG AAT GTG TTT TTT AAT GCG ATG AAT AAG GCT TTA GAG AAT AAG AAT 576
   Gly Asn Val Phe Phe Asn Ala Met Asn Lys Ala Leu Glu Asn Lys Asn
   180 185 190
   GGA ACT AGT AGT GCT AGT GGA ACT AGT GGT GCG ACT GGT TCA GAT GGT 624
   Gly Thr Ser Ser Ala Ser Gly Thr Ser Gly Ala Thr Gly Ser Asp Gly
   195 200 205
   CAA ACT TAC TCC ACA CAA GCT ATC CAA TAC CTT CAA GGC CAA CAA AAT 672
   Gln Thr Tyr Ser Thr Gln Ala Ile Gln Tyr Leu Gln Gly Gln Gln Asn
   210 215 220
   ATC TTA AAT AAC GCA GCG AAC TTG CTC AAG CAA GAT GAA TTG CTC TTA 720
   Ile Leu Asn Asn Ala Ala Asn Leu Leu Lys Gln Asp Glu Leu Leu Leu
   225 230 235 240
   GAA GCT TTC AAC TCT GCC GTA GCC GCC AAC ATT GGG AAT AAG GAA TTC 768
   Glu Ala Phe Asn Ser Ala Val Ala Ala Asn Ile Gly Asn Lys Glu Phe
   245 250 255
   AAT TCA GCC GCT TTT ACA GGT TTG GTG CAA GGC ATT ATT GAT CAA TCT 816
   Asn Ser Ala Ala Phe Thr Gly Leu Val Gln Gly Ile Ile Asp Gln Ser
   260 265 270
   CAA GCG GTT TAT AAC GAG CTC ACT AAA AAC ACC ATT AGC GGG AGT GCG 864
   Gln Ala Val Tyr Asn Glu Leu Thr Lys Asn Thr Ile Ser Gly Ser Ala
   275 280 285
   GTT ATT AGC GCT GGG ATA AAC TCC AAC CAA GCT AAC GCT GTG CAA GGG 912
   Val Ile Ser Ala Gly Ile Asn Ser Asn Gln Ala Asn Ala Val Gln Gly
   290 295 300
   CGC GCT AGT CAG CTC CCT AAC GCT CTT TAT AAC GCG CAA GTA ACT TTG      960
   Arg Ala Ser Gln Leu Pro Asn Ala Leu Tyr Asn Ala Gln Val Thr Leu
   305                 310                 315                 320
   GAT AAA ATC AAT GCG CTC AAT AAT CAA GTG AGA AGC ATG CCT TAC TTG     1008
   Asp Lys Ile Asn Ala Leu Asn Asn Gln Val Arg Ser Met Pro Tyr Leu
                   325                 330                 335
   CCC CAA TTC AGA GCC GGG AAC AGC CGT TCA ACG AAT ATT TTA AAC GGG     1056
   Pro Gln Phe Arg Ala Gly Asn Ser Arg Ser Thr Asn Ile Leu Asn Gly
               340                 345                 350
   TTT TAC ACC AAA ATA GGC TAT AAG CAA TTC TTC GGG AAG AAA AGG AAT     1104
   Phe Tyr Thr Lys Ile Gly Tyr Lys Gln Phe Phe Gly Lys Lys Arg Asn
           355                 360                 365
   ATC GGT TTG CGC TAT TAT GGT TTC TTT TCT TAT AAC GGA GCG AGC GTG     1152
   Ile Gly Leu Arg Tyr Tyr Gly Phe Phe Ser Tyr Asn Gly Ala Ser Val
       370                 375                 380
   GGC TTT AGA TCC ACT CAA AAT AAT GTA GGG TTA TAC ACT TAT GGG GTG     1200
   Gly Phe Arg Ser Thr Gln Asn Asn Val Gly Leu Tyr Thr Tyr Gly Val
   385                 390                 395                 400
   GGG ACT GAT GTG TTG TAT AAC ATC TTT AGC CGC TCC TAT CAA AAC CGC     1248
   Gly Thr Asp Val Leu Tyr Asn Ile Phe Ser Arg Ser Tyr Gln Asn Arg
                   405                 410                 415
   TCT GTG GAT ATG GGC TTT TTT AGC GGT ATC CAA TTA GCC GGT GAG ACC     1296
   Ser Val Asp Met Gly Phe Phe Ser Gly Ile Gln Leu Ala Gly Glu Thr
               420                 425                 430
   TTC CAA TCC ACG CTC AGA GAT GAC CCC AAT GTG AAA TTG CAT GGG AAA     1344
   Phe Gln Ser Thr Leu Arg Asp Asp Pro Asn Val Lys Leu His Gly Lys
           435                 440                 445
   ATC AAT AAC ACG CAC TTC CAG TTC CTC TTT GAC TTC GGT ATG AGG ATG     1392
   Ile Asn Asn Thr His Phe Gln Phe Leu Phe Asp Phe Gly Met Arg Met
       450                 455                 460
   AAC TTC GGT AAG TTG GAC GGG AAA TCC AAC CGC CAC AAC CAG CAC ACG     1440
   Asn Phe Gly Lys Leu Asp Gly Lys Ser Asn Arg His Asn Gln His Thr
   465                 470                 475                 480
   GTG GAA TTT GGC GTA GTG GTG CCT ACG ATT TAT AAC ACT TAT TAC AAA     1488
   Val Glu Phe Gly Val Val Val Pro Thr Ile Tyr Asn Thr Tyr Tyr Lys
                   485                 490                 495
   TCA GCA GGG ACT ACC GTG AAG TAT TTC CGT CCT TAT AGC GTT TAT TGG     1536
   Ser Ala Gly Thr Thr Val Lys Tyr Phe Arg Pro Tyr Ser Val Tyr Trp
               500                 505                 510
   TCT TAT GGG TAT TCA TTC TAA                                         1557
   Ser Tyr Gly Tyr Ser Phe
           515
(2) ANGABEN ZU SEQ ID NO: 2:
   (i) SEQUENZKENNZEICHEN:
      (A) LÄNGE: 518 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Ile Lys Lys Asn Arg Thr Leu Phe Leu Ser Leu Ala Leu Cys Ala
    1               5                  10                  15
   Ser Ile Ser Tyr Ala Glu Asp Asp Gly Gly Phe Phe Thr Val Gly Tyr
               20                  25                  30
   Gln Leu Gly Gln Val Met Gln Asp Val Gln Asn Pro Gly Gly Ala Lys
           35                  40                  45
   Ser Asp Glu Leu Ala Arg Glu Leu Asn Ala Asp Val Thr Asn Asn Ile
       50                  55                  60
   Leu Asn Asn Asn Thr Gly Gly Asn Ile Ala Gly Ala Leu Ser Asn Ala
   65                  70                  75                  80
   Phe Ser Gln Tyr Leu Tyr Ser Leu Leu Gly Ala Tyr Pro Thr Lys Leu
                   85                  90                  95
   Asn Gly Ser Asp Val Ser Ala Asn Ala Leu Leu Ser Gly Ala Val Gly
               100                 105                 110
   Ser Gly Thr Cys Ala Ala Ala Gly Thr Ala Gly Gly Thr Ser Leu Asn
           115                 120                 125
   Thr Gln Ser Thr Cys Thr Val Ala Gly Tyr Tyr Trp Leu Pro Ser Leu
       130                 135                 140
   Thr Asp Arg Ile Leu Ser Thr Ile Gly Ser Gln Thr Asn Tyr Gly Thr
   145                 150                 155                 160
   Asn Thr Asn Phe Pro Asn Met Gln Gln Gln Leu Thr Tyr Leu Asn Ala
                   165                 170                 175
   Gly Asn Val Phe Phe Asn Ala Met Asn Lys Ala Leu Glu Asn Lys Asn
               180                 185                 190
   Gly Thr Ser Ser Ala Ser Gly Thr Ser Gly Ala Thr Gly Ser Asp Gly
           195                 200                 205
   Gln Thr Tyr Ser Thr Gln Ala Ile Gln Tyr Leu Gln Gly Gln Gln Asn
       210                 215                 220
   Ile Leu Asn Asn Ala Ala Asn Leu Leu Lys Gln Asp Glu Leu Leu Leu
   225                 230                 235                 240
   Glu Ala Phe Asn Ser Ala Val Ala Ala Asn Ile Gly Asn Lys Glu Phe
                   245                 250                 255
   Asn Ser Ala Ala Phe Thr Gly Leu Val Gln Gly Ile Ile Asp Gln Ser
               260                 265                 270
   Gln Ala Val Tyr Asn Glu Leu Thr Lys Asn Thr Ile Ser Gly Ser Ala
           275                 280                 285
   Val Ile Ser Ala Gly Ile Asn Ser Asn Gln Ala Asn Ala Val Gln Gly
       290                 295                 300
   Arg Ala Ser Gln Leu Pro Asn Ala Leu Tyr Asn Ala Gln Val Thr Leu
   305                 310                 315                 320
   Asp Lys Ile Asn Ala Leu Asn Asn Gln Val Arg Ser Met Pro Tyr Leu
                   325                 330                 335
   Pro Gln Phe Arg Ala Gly Asn Ser Arg Ser Thr Asn Ile Leu Asn Gly
               340                 345                 350
   Phe Tyr Thr Lys Ile Gly Tyr Lys Gln Phe Phe Gly Lys Lys Arg Asn
           355                 360                 365
   Ile Gly Leu Arg Tyr Tyr Gly Phe Phe Ser Tyr Asn Gly Ala Ser Val
       370                 375                 380
   Gly Phe Arg Ser Thr Gln Asn Asn Val Gly Leu Tyr Thr Tyr Gly Val
   385                 390                 395                     400
   Gly Thr Asp Val Leu Tyr Asn Ile Phe Ser Arg Ser Tyr Gln Asn Arg
                   405                 410                 415
   Ser Val Asp Met Gly Phe Phe Ser Gly Ile Gln Leu Ala Gly Glu Thr
               420                 425                 430
   Phe Gln Ser Thr Leu Arg Asp Asp Pro Asn Val Lys Leu His Gly Lys
           435                 440                 445
   Ile Asn Asn Thr His Phe Gln Phe Leu Phe Asp Phe Gly Met Arg Met
       450                 455                 460
   Asn Phe Gly Lys Leu Asp Gly Lys Ser Asn Arg His Asn Gln His Thr
   465                 470                 475                     480
   Val Glu Phe Gly Val Val Val Pro Thr Ile Tyr Asn Thr Tyr Tyr Lys
                   485                 490                 495
   Ser Ala Gly Thr Thr Val Lys Tyr Phe Arg Pro Tyr Ser Val Tyr Trp
               500                 505                 510
   Ser Tyr Gly Tyr Ser Phe
           515

## Beschreibung

Die vorliegende Erfindung betrifft ein Verfahren zur Identifizierung sekretorischer Gene und insbesondere von Adhärenzgenen aus Helicobacter pylori. Weiterhin betrifft die Erfindung eine zur Identifizierung von sekretorischen Genen aus H. pylori geeignete Genbank, aus dieser Genbank erhältliche Klone, Polynucleotide und Polypeptide, insbesondere das alpA-Gen aus Helicobacter pylori und das davon codierte Polypeptid. Diese Polynucleotide und Polypeptide können zur Diagnose, Prävention und Behandlung einer Helicobacter-Infektion eingesetzt werden. Daher betrifft die vorliegende Erfindung auch pharmazeutische Zusammensetzungen.

Das Auftreten von spiralförmigen Bakterien in der menschlichen Magenschleimhaut ist seit langem bekannt (Bizzozero, 1893). Die Tatsache, daß es sich dabei um pathogene Keime handelt, wurde jedoch erst mit der erfolgreichen Isolierung und Kultivierung dieses Bakteriums durch Narshall und Warren (Warren and Marshall, 1983; Marshall et al., 1984) aus der Magenschleimhaut eines Patienten mit einem Magengeschwür (Ulcus ventriculi) realisiert. Wie erste Analysen ergaben, handelte es sich bei den isolierten Mikroorganismen um gramnegative, spiralförmige Bakterien mit extrem hoher Beweglichkeit und der ungewöhnlichen Fähigkeit im stark sauren Milieu (bis ca. pH 1,5) zu überleben. Ursprünglich als Campylobacter pylori bezeichnet, wurden die Keime schließlich aufgrund biochemischer und morphologischer Eigenschaften in die neu gegründete Gattung "Helicobacter" eingruppiert (Goodwin et al., 1989).

Schon innerhalb weniger Jahre wurde die Bedeutung der Helicobacter pylori-Infektion und die Tragweite dieser Entdeckung klar. Epidemiologische Untersuchungen von Taylor und Blaser (1991) zeigten, daß die H. pylori Infektion weltweit auftritt, und daß ca. 50 % der Bevölkerung mit diesem Bakterium infiziert sind, wobei die Infektionsrate in den Entwicklungsländern höher ist als in industrialisierten Ländern. Ferner beobachtet man, daß die Wahrscheinlichkeit einer chronischen H. pylori-Infektion mit steigendem Lebensalter drastisch zunimmt. Damit zählt die H. pylori-Infektion zu den häufigsten chronischen bakteriellen Infektionen des Menschen.

Heute weiß man, daß die Infektion zwangsläufig zur Auslösung einer bakteriellen Gastritis (Typ-B Gastritis) beim Menschen führt. Ferner geht man davon aus, daß H. pylori auch eine ursächliche Rolle bei der Entstehung von Magen- und Zwölffingerdarmgeschwüren (Ulcus ventriculi und Ulcus duodeni) sowie bei einigen Formen des Magenkarzinoms (Adenokarzinom) spielt (Lee et al., 1993; Solnick and Tompkins, 1993). Auch die seltener auftretenden MALT (Mucosa Associated Lymphoid Tissue) Lymphome des Magens, die als Vorstufen von B-Zell-Tumoren des Immunsystems angesehen werden, sind vermutlich eine Folge der H. pylori-Infektion. Eine antibakterielle Behandlung entsprechender Patienten mit erfolgreicher Eradikation (totaler Elimination) von H. pylori führt sowohl zu einer Abheilung von Magengeschwüren als auch von "low grade" MALT Lymphomen (Sipponen and Hyvärinen, 1993; Isaacson and Spencer, 1993; Stolte and Eidt, 1993).

Eine Folge der Langzeitinfektion mit H. pylori ist die atrophische Gastritis, eine Degeneration der Schleim-, Säure- oder Pepsinproduzierenden Zellen des Magenepithels, die als eine präkanzeröse Läsion angesehen werden muß. Nach einer Statistik der 1980 weltweit am häufigsten aufgetretenen Krebsarten steht das Magenkarzinom an zweiter Stelle, allerdings mit rückläufiger Tendenz (Parkin et al., 1988). Zwei Studien zeigten kürzlich eine statistisch signifikante Korrelation zwischen der H.pylori- Infektion und dem Auftreten des Magenkarzinoms (intestinaler Typ); beide kamen zu dem Schluß, daß ca. 60 % aller auftretenden Magenkarzinome wahrscheinlich auf eine H. pylori- Infektion zurückzuführen sind (Parsonnet et al., 1991; Nomura et al., 1991). Weihterhin zeigen Untersuchungen von Sipponen (1992), daß in vielen industrialisierten Ländern mehr als 20 % der Infizierten im Laufe ihres Lebens an einem Ulkus des Magens oder des Duodenums erkranken, während bei Personen mit normaler Magenmukosa dieses Risiko vernachlässigbar gering ist. Dies bedeutet, daß diese häufigen gastroduodenalen Erkrankungen als Infektionskrankheiten betrachtet und entsprechend behandelt werden müssen (Alper, 1993). Eine Behandlung, die eine bereits bestehende chronische H. pylori-Infektion eliminiert, führt zur Abheilung einer Gastritis, eines Magen- bzw. Zwölffingerdarmgeschwürs oder eines MALT-Lymphoms. Damit kann eine prophylaktische Behandlung, die eine H. pylori-Infektion verhindert (z. B. Impfung) sowie eine Behandlung die eine bereits bestehende H. pylori-Infektion eliminiert, zur Behandlung dieser häufigen gastroduodenalen Erkrankungen eingesetzt werden.

Neben einigen höheren Primaten war der Mensch bisher als einziger natürlicher Wirt für H. pylori bekannt. Der relativ neue Befund, daß auch die Hauskatze mit H. pylori infiziert sein kann, wirft ein neues Licht auf die Frage der Übertragung und eines möglichen Reservoirs für diese Bakterien außerhalb des menschlichen Organismus. Die gelegentlich erfolgreiche Anzüchtung von H. pylori aus dem Stuhl infizierter Personen und die Fähigkeit der Bakterien über Monate im Wasser zu überleben, unterstützen die Hypothese einer fäkal-oralen Übertragung. Auch die direkte oral-orale Transmission wird aufgrund von Familienstudien als wahrscheinlich angesehen. Die Infektion erfolgt meist im Kindesalter innerhalb der Familie, wobei enge räumliche Lebensverhältnisse und geringer Hygienestandard positiv mit der Häufigkeit der Infektion korrelieren.

Nach der oralen Aufnahme gelangen die Bakterien zunächst in das extrem saure Magenlumen (pH 1 - 2). Dort wird durch die Produktion des Enzyms Urease, das zur Spaltung des vorhandenen Harnstoffs und damit zur lokalen Neutralisierung des sauren pH-Wertes im Magen führt, das Überleben der Bakterien ermöglicht. Mittels chemotaktischer Orientierung und flagellenabhängiger Motilität bewegen sich die Keime dann in die Bicarbonat-gepufferte Schleimschicht der Antrumregion des Magens, ihrem eigentlichen natürlichen Habitat. Dort befinden sie sich in einer einzigartigen ökologischen Nische, die aufgrund der Säurebarriere nur für wenige konkurrierende Bakterienarten zugänglich ist. Vermutlich orientieren sich die Keime an dem pH-Gradienten zwischen Lumen (pH 1-2) und Epithelzelloberfläche (pH 6-7), um zum Epithel zu gelangen. Durch ihre spiralige Form, ihre Beweglichkeit im viskosen Schleim, die Produktion von Mukus-modifizierenden Enzymen und schließlich durch eine mikroaerophile Lebensweise sind diese Keime optimal an die Lebensbedingungen in diesem Habitat angepaßt.

Sie halten sich meist in den tiefen Krypten der Antrumregion auf, wo sie vor äußeren Einflüssen wie z. B. Säure, Pepsin, aber auch vor Medikamenten zu ihrer Eradikation wie z. B. Antibiotika geschützt sind. Ein Teil der Population (ca. 20 %) ist eng mit Epithelzellen assoziiert, vor allem mit Schleim-produzierenden Zellen. Unter der Voraussetzung einer gastralen Metaplasie, d. h. der Säure-induzierten Ausbildung von gastralem Epithel im Duodenum, kommt es auch zur Kolonisierung metaplastischer Areale im Duodenum, wodurch die Voraussetzungen zur Entstehung des Zwölffingerdarm-Geschwürs (Ulcus duodeni) geschaffen sind. Durch ihre Fähigkeit zur Adhärenz wird vermutlich eine komplette Ausscheidung der Helicobacter mit dem abgestoßenen Schleim verhindert, so daß die Bakterien für Jahre, Jahrzehnte oder gar lebenslang persistieren können (chronische Infektion).

Bevor die Existenz und die Bedeutung des H. pylori für die Ulkuserkrankungen bekannt waren, wurden diese durch sog. Antazida, oder H₂-Rezeptorantagonisten behandelt. Dabei handelt es sich um Substanzen, welche die Säuresekretion der Magenparietalzellen inhibieren. Unter dem Einfluß dieser Arzneimittel kommt es meist zur Abheilung von Geschwüren, da jedoch eine der Ursachen dieser Geschwüre, nämlich die H. pylori-Infektion, damit nicht eliminiert wird, kommt es in den meisten Fällen nach kurzer Zeit zu einem erneuten Auftreten der Ulzeration (Rezidiv).

Eine weitere, häufig angewandte Therapie bei Ulzerationen ist die Wismut-Behandlung. Verschiedene Wismutsalze (CBS, BSS) haben einen bakteriziden Effekt auf H. pylori. Eine totale Eradikation des Keims wird jedoch nur in 8 - 32 % der Fälle erreicht. Die Behandlung führt anscheinend zu einer vorübergehenden Suppression des Keims, aber nach Absetzen der Behandlung kommt es in den meisten Fällen wieder zum Aufflackern der Infektion. Eine längerdauernde Therapie mit hohen Dosen führt zu einer Akkumulation der Substanz in Leber, Niere und im Nervensystem und hat beträchtliche neurologische Nebenwirkungen (Malfertheiner, 1994).

Seit der Erkenntnis, daß es sich bei den gastroduodenalen Ulkuserkrankungen um Infektionskrankheiten handelt, ist ein Ziel der Behandlung die Eradikation der Erreger durch Antibiotika. Die Monotherapie mit verschiedenen Antibiotika (Amoxicillin, Nitrofuran, Furazolidin, Erythromycin u. a.) stellte sich jedoch als nicht zufriedenstellend heraus, da es auch hier nur bei 0 - 15 % der Fälle zur Eradikation der Keime kommt. Die erfolgreichste Behandlung wird zur Zeit durch eine Kombination eines Säureblockers (Ompeprazol) mit einem Antibiotikum (Amoxicillin) erreicht, die zu Eradikationsraten bis zu 80 % führen kann (Malfertheiner, 1994). Auf Dauer ist eine Antibiotika-Behandlung zur Eliminierung von H. pylori jedoch nicht erfolgversprechend, da mit einer raschen Resistenzentwicklung der Bakterien gegen Antibiotika gerechnet werden muß.

Daher besteht ein Bedürfnis nach neuen Therapieformen für die Bekämpfung einer H. pylori-Infektion, insbesondere nach Impfstoffen, die spezifisch gegen Virulenzfaktoren von H. pylori gerichtet sind. Als Virulenzfaktoren bezeichnet man die Eigenschaften eines pathogenen Bakteriums, die ihm die Fähigkeit verleihen, eine bestimmte ökologische Nische im Körper des Wirts zu kolonisieren und sich dort trotz der Immunantwort und der unspezifischen Abwehrmechanismen des Wirtsorganismus zu vermehren. Kenntnisse über Virulenzfaktoren helfen somit den Ablauf und die Mechanismen einer Infektionskrankheit besser zu verstehen. Die wichtigsten bisher untersuchten Virulenzfaktoren von H. pylori sind die Urease, die Flagellen, die Adhäsine und die Produktion eines Cytotoxins.

Die Urease, ein Enzym auf der Oberfläche der Bakterien, besteht aus 2 Untereinheiten (UreA, 26 kDa, UreB, 66 kDa), die bis zu 5 % des gesamten bakteriellen Proteins ausmachen. Die Urease spaltet den im Magensaft in geringer Konzentration vorkommenden Harnstoff in Ammoniak und Kohlendioxid. Nach der gängigen Modellvorstellung umgibt sich das Bakterium mit einer Wolke von Ammoniak, was zur lokalen Neutralisierung der Säure des Magensaftes führt. Die außerordentlich hohe Beweglichkeit der Bakterien kann auf das Vorhandensein von polaren Flagellen zurückgeführt werden, die es dem Bakterium erlauben, sich im viskosen Mukus der Magenschleimhaut zu bewegen und dadurch die Epithelzellschicht zu erreichen. Sowohl das Urease-Gencluster (ureA - ureH) als auch die Gene zur Ausbildung der Flagellen (flaA, flaB) wurden in E. coli kloniert und sequenziert und es wurden isogene Mutanten hergestellt.

Ungefähr 50 - 60 % aller isolierten H. pylori-Stämme produzieren ein 87 kDa Protein, das sogenannte vakuolisierende Cytotoxin, das bei in vitro Zellkulturen die Ausbildung von cytoplasmatischen Vakuolen bewirkt. Auch das vacA-Gen, das für das Cytotoxin von H. pylori kodiert, wurde inzwischen kloniert und genetisch charakterisiert (Schmitt W. und Haas R. (1994), Mol. Microbiol. 12 (2), 307-319). Es wird ferner vermutet, daß die Cytotoxinproduzierenden Stämme ein höheres pathogenes Potential besitzen, als Stämme, die dieses Toxin nicht produzieren. Weiterhin wurde eine positive Korrelation zwischen der Produktion des Cytotoxins und der Ausbildung von Magengeschwüren gefunden.

Untersuchungen zur Adhärenz von H. pylori an Epithelzellinien in vitro zeigen, daß die Bakterien an viele Zellinien von unterschiedlichen Geweben binden können. Im Wirtsorganismus dagegen zeigt H. pylori eine sehr ausgeprägte Spezies- und gewebeselektive Adhärenz (Tropismus). So findet man die Bakterien nur an Epithelzellen gebunden, die dem gastralen Typ von Epithelzellen angehören. Diese Selektivität wird durch eine spezifische Interaktion zwischen einem bakteriellen Adhäsin und einem spezifischen zellulären Rezeptor erklärt.

Es wurden bisher mehrere potentielle Adhäsine von H. pylori beschrieben und ein Gen (hpaA), das für ein sogenanntes N-Acetyl-Neuraminyllactose-bindendes Hemagglutinin kodiert, wurde kloniert und sequenziert (Evans et al., 1993). Dabei handelt es sich um ein Protein, das einen sialinsäurehaltigen Rezeptor auf den Epithelzellen erkennen soll. Die Bedeutung dieses Adhäsins für die H. pylori-Infektion ist jedoch umstritten. Andere potentielle Adhäsine sind entweder nur durch ihr Molekulargewicht oder ihre Rezeptorbindungsspezifität charakterisiert. Dazu zählt ein 63 kDa Protein, das zum Exoenzym S von Pseudomonas aeruginosa, einem Adhäsin mit ADP-Ribosyltransferase-Aktivität, homolog zu sein scheint. Ferner vermutet man ein noch nicht identifiziertes Adhäsin, welches eine spezifische Bindung an das Lewis^{b}-Blutgruppenantigen der Magenepithelzellen vermittelt (Falk et al., 1993; Borén et al., 1993).

Transposons sind ein seit einiger Zeit verstärkt zur Klonierung und für Mutagenesezwecke eingsetztes Werkzeug. So offenbaren z.B. Tadayyon und Broome-Smith (Gene 111 (1993), 21-26) den Einsatz des blaM Gens zur Mutagenese und Selektion von exportierten Genen im Transposon Tu5, welches allerdings bekanntermaßen in Helicobacter nicht funktionstüchtig ist.

Eine Publikation von Haas et al. (Gene 130 (1993), 23-31) beschreibt die Verwendung des Minitransposons TnMax für die Analyse einzelner klonierter Gene und für die Transposon-Shuttle-Mutagenese. Kahrs et al. (Mol. Microbiol. (1994), 12 (5), 819-831) offenbaren ein Verfahren zur Isolierung von Neisseria gonorrhoeae Mutanten. Das verwendete Plasmid zum Anlegen der Genbank ist allerdings kein dem hier beschriebenen Miniplasmid vergleichbarer Vektor. Weitere Transposons und Minitransposons sind u.a. in Reidl und Mekalanos (Mol. Microbiol. (1992), 6 (13), 1829-1839), U.S. Patent Nr. 4,914,025 und FR 2 693 475 beschrieben. Auch Fusionsproteine sind im Stand der Technik bekannt (Broome-Smith und Spratt: Gene 49 (1986), 341-349).

Die Infektion mit H. pylori führt zu einer chronischen Entzündungsreaktion der Magenmukosa (Gastritis). Ferner wird eine spezifische systemische Immunantwort gegen H. pylori-Antigene induziert, die Bildung von sekretorischen Antikörpern im Magen (sIgA) ist allerdings noch nicht eindeutig geklärt. Durch die Entzündung befinden sich verschiedene Immunzellen in der Magenmukosa und Submukosa, z. B. polymorphkernige Leukozyten, Monozyten, Makrophagen, Lymphozyten und Plasmazellen (Blaser, 1992). Weiterhin aktiviert H. pylori sowohl Neutrophile als auch Monozyten und Makrophagen in vitro (Mai et al., 1991). In vitro zeigen Versuche mit spezifischen Antikörpern und Komplement eine rasche Inaktivierung von H. pylori durch Neutrophile. In der in vivo Situation führen diese Mechanismen jedoch nicht zur Inaktivierung der pathogenen Bakterien. Wie H. pylori über lange Zeit im Wirt überlebt obwohl dieser die obengenannten Abwehrmechanismen aktiviert, ist unklar.

Der Wirt ist nicht in der Lage, unter natürlichen Bedingungen mit der H. pylori-Infektion fertig zu werden. Um so überraschender war es deshalb, daß die Urease, ein essentieller Virulenzfaktor von H. pylori (s. o.), ein hohes Potential als Vakzine besitzt (US-Patentanmeldung USSN 07/970,996 "Urease-based Vaccine against Helicobacter Infection").

Im Helicobacter felis / Maus Modell (H. felis ist eine Helicobacter Spezies, die natürlicherweise im Magen der Katze kolonisiert und auch die Maus infizieren kann) konnte gezeigt werden, daß die H. pylori Urease, bzw die rekombinante Urease B Untereinheit (rUreB) bei oraler Vakzinierung, sowohl die Maus vor einer H. felis-Infektion schützen kann (präventive Vakzine), als auch eine bereits bestehende Infektion eliminieren kann (therapeutische Vakzine) (Michetti et al., 1994; Corthesy-Theulaz et al., Gastroenterol., im Druck). Entscheidend bei der oralen Vakzinierung war der Einsatz von Adjuvantien wie z. B. Choleratoxin, das u. a. zur Konversion der Immunreaktion von der Produktion von systemischen Antikörpern zu sekretorischen Antikörpern wichtig zu sein scheint.

Die der vorliegenden Erfindung zugrundeliegende Aufgabe bestand darin, ein Verfahren bereitzustellen, mit dem sekretorische Gene aus Helicobacter pylori, die potentielle Kandidaten für Impfstoffe sind, auf einfache und schnelle Weise identifiziert werden können.

Diese Aufgabe wird gelöst durch ein Verfahren zur Identifizierung sekretorischer Gene aus Helicobacter pylori, wobei man
(a) eine Genbank von H. pylori DNA in einem Minimal-Plasmidvektor, der im wesentlichen nur aus zur Replikation, Selektion und Klonierung notwendigen Elementen besteht und eine Selektion gegen eine Transposon-Insertionin die Vektor-DNA erlaubt, in einem Wirtsorganismus anlegt, der ein induzierbares Transposon gekoppelt mit einem Marker für sekretorische Aktivität enthält,
(b) die Insertion des Transposons in die H. pylori-DNA induziert und
(c) mit Hilfe des Markers eine Selektion auf Klone durchführt, die ein sekretorisches Gen enthalten.

(Michetti et al., 1994; Corthesy-Theulaz et al., Gastroenterol., im Druck) . Entscheidend bei der oralen Vakzinierung war der Einsatz von Adjuvantien wie z. B. Choleratoxin, das u. a. zur Konversion der Immunreaktion von der Produktion von systemischen Antikörpern zu sekretorischen Antikörpern wichtig zu sein scheint.

Die der vorliegenden Erfindung zugrundeliegende Aufgabe bestand darin, ein Verfahren bereitzustellen, mit dem sekretorische Gene aus Helicobacter pylori, die potentielle Kandidaten für Impfstoffe sind, auf einfache und schnelle Weise identifiziert werden können.

Diese Aufgabe wird gelöst durch ein Verfahren zur Identifizierung sekretorischer Gene aus Helicobacter pylori, wobei man
(a) eine Genbank von H. pylori DNA in einem Wirtsorganismus anlegt, der ein induzierbares Transposon gekoppelt mit einem Marker für sekretorische Aktivität enthält,
(b) die Insertion des Transposons in die H. pylori-DNA induziert und
(c) mit Hilfe des Markers eine Selektion auf Klone durchführt, die ein sekretorisches Gen enthalten.

Der Begriff "sekretorisches Gen" oder "Gen mit sekretorischer Aktivität" soll ein Gen bezeichnen, welches für sekretorisches Polypeptid, d. h. für ein aus dem Cytoplasma exportiertes Polypeptid codiert.

Das erfindungsgemäße Verfahren ist insbesondere zur Identifizierung von Adhärenzgenen aus H. pylori geeignet, indem man weiterhin
(d) eine Retransformation von H. pylori mit der DNA von Klonen der Genbank, vorzugsweise mit Klonen, die Gene mit sekretorischer Aktivität enthalten, durchführt, wobei durch Integration der DNA in das Chromosom isogene H. pylori-Mutantenstämme erzeugt werden und
(e) eine Selektion auf Adhärenz-defiziente H. pylori-Mutantenstämme durchführt.

Durch das erfindungsgemäße Verfahren ist es möglich, Adhärenzgene und die davon codierten Adhärenzproteine (Adhäsine) aus H. pylori zu identifizieren und isolieren, die für die spezifische Wechselwirkung der Bakterien mit den Epithelzellen der Magenmucosa verantwortlich sind. Da Adhäsine aus H. pylori meist nur in sehr geringen Mengen produziert werden, ist deren Identifizierung und Isolierung auf biochemischem Wege meist mit erheblichen Schwierigkeiten verbunden. Aufgrund der Tendenz von H. pylori zur spontanen Autolyse werden darüber hinaus cytoplasmatische Proteine freigesetzt und binden auf der Bakterienoberfläche. Bei einer biochemischen Aufreinigung wurden solche Proteine in der Vergangenheit irrtümlich als Adhäsine identifiziert (Doig et al, 1992; Doig et al, 1993).

Durch das erfindungsgemäße Verfahren ist eine schnelle und einfache Identifizierung von Adhäsinen möglich. Dabei werden einzelne Gene im Chromosom von H. pylori mit Hilfe eine Transposons inaktiviert und Mutanten hergestellt, die in unterschiedlichen chromosomalen Genen defekt sind. Aus diesen unabhängigen Defektmutanten können durch gezielte Selektion diejenigen Mutanten ausgewählt werden, die in Adhäsingenen inaktiviert sind und damit nicht mehr zur Bindung an Rezeptoren der entsprechenden Zielzellen in der Lage sind.

Der erste Schritt des erfindungsgemäßen Verfahrens umfaßt das Anlegen einer Genbank von H. pylori-DNA in einem Wirtsorganismus, vorzugsweise einer Plasmidgenbank in einem prokaryontischen Wirtsorganismus, wie etwa E. coli.

Anschließend werden die klonierten H. pylori-Gene mit Hilfe eines Transposons mutagenisiert, welches mit einem Marker für sekretorische Aktivität gekoppelt ist.

Ein besonders bevorzugtes Transposon ist das Transposon TnMax9, das bei der deutschen Sammlung von Mikroorganismen und Zellkulturen GmbH (DSM), Mascheroderweg 1b, DE-38124 Braunschweig im E.coli Stamm E 181 unter dem Aktenzeichen DSM 10008 gemäß den Vorschriften des Budapester Vertrags am 26.05.95 hinterlegt wurde.

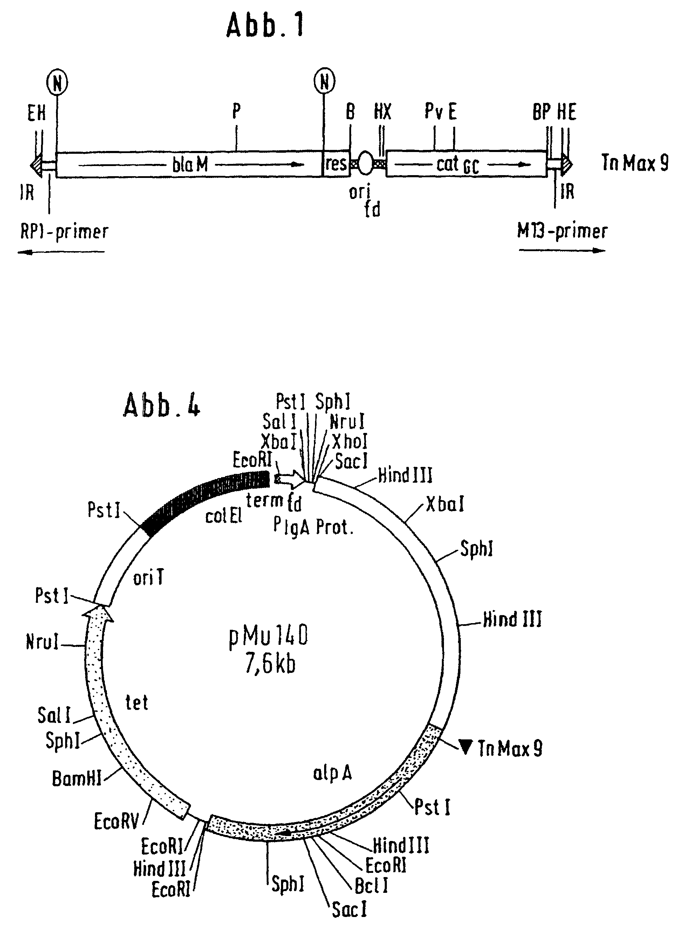

Eine schematische Darstellung von TnMax9 ist in Abb. 1 gezeigt. TnMax9 trägt direkt im Anschluß an die das eigentliche Transposon begrenzenden inverted repeats (IR) die Kopie eines β-Lactamasegens ohne Promotor und Signalsequenz (blaM) als Marker (Tadayyon und Broome-Smith, 1982). Das blaM-Gen fusioniert nach Insertion von TnMax9 mit dem Zielgen und bei Expression des inaktivierten Zielgens wird ein Fusionsprotein zwischen dem Zielgen X und BlaM erzeugt, falls das Transposon in der korrekten Orientierung und im korrekten Leserahmen bezüglich des Gens X insertiert. Sofern das inaktivierte Gen X für ein sekretorisches Protein, insbesondere für ein durch den Sec-abhängigen Transportweg exportiertes Protein codiert (Pugsley, 1993), wird das Fusionsprotein durch die Cytoplasmamembran der E. coli-Wirtszelle in das Periplasma transportiert. Dort entfaltet das Fusionsprotein seine Aktivität (Spaltung von β-Lactam-Antibiotika) und vermittelt somit eine Resistenz des entsprechenden E. coli-Klons gegen das Antibiotikum Ampicillin.

Handelt es sich bei dem Gen X jedoch um ein Gen für ein cytoplasmatisches Protein, kann es je nach Insertion (Orientierung, Raster) ebenfalls zu einem Fusionsprotein kommen, dieses weist jedoch keine β-Lactamase-Aktivität auf, da die β-Lactamase nur in periplasmatischer Umgebung, jedoch nicht im Cytoplasma enzymatisch aktiv ist. Damit können mit dieser Methode spezifisch Mutanten in Genen identifiziert werden, die für exportierte Proteine kodieren, indem die Mutanten nach der Transposon-Mutagenese in E. coli auf β-Lactamase-Aktivität gescreent werden. Da auch die gesuchten Adhäsingene von H. pylori Exportproteine sein müssen, kann eine spezielle Kollektion von H. pylori Transposon-Mutanten in Genen, die für exportierte Proteine kodieren, die Anzahl der zu testenden Mutanten stark verringern. Auf diese Weise wird durch das erfindungsgemäße Verfahren eine stark angereicherte Mutantenbank bereitgestellt, mit der ein in der Laborpraxis nur sehr schwer durchführbares Screening einer üblichen Mutantenbank mit ca. 2000 - 4000 Klonen vermieden werden kann. Die Mehrzahl der chromosomalen Gene eines Bakteriums, die für cytoplasmatische Proteine kodieren, kann durch den auf die Identifizierung von sekretorischen Genen gerichteten Selektionsschritt von vornherein ausgeschaltet werden. So kann die Anzahl der H. pylori-Mutanten, welche auf den Verlust ihrer Adhärenzfähigkeit an bestimmte Targetzellen getestet werden müssen, auf ein in der Praxis vertretbares Maß reduziert werden.

Das Screening von bakteriellen Adhärenz-Mutanten kann auf verschiedenen Ebenen durchgeführt werden. Meist werden dafür Epithelzellinien verwendet, an die die Bakterien spezifisch binden. Für H. pylori bieten sich spezielle Magenkarzinomzellinien an, wie z. B. die KatoIII Zellinie (ATCC HTB 103), die bereits von verschiedenen Autoren als Adhärenzmodell beschrieben wurde (Clyne and Drumm, 1993). Zellinien sind zwar relativ einfach zu handhaben und meist in großen Mengen in vitro zu züchten, durch die Immortalisierung der Zellen können jedoch Veränderungen in der quantitativen und qualitativen Expression von Oberflächenproteinen, wie z. B. von Rezeptoren, grundsätzlich nicht ausgeschlossen werden. Eine Alternative dazu stellen deshalb Gewebemodelle dar. Dabei werden von fixiertem Gewebe ultradünne Schnitte hergestellt (Mikrotom), die nach Absättigung mit den Bakterien inkubiert werden. Bakterien, die nicht an die Rezeptoren binden, werden abgewaschen, und die gebundenen Bakterien können mit Farbstoffen (Fluoreszenzfarbstoffe oder spezielle Bakterienfarbstoffe) sichtbar gemacht werden. Als dritte Stufe bieten sich Tiermodelle an. Dabei handelt es sich um in vivo Versuche, die nur durchgeführt werden können, wenn entsprechende Tiermodelle für die Mikroorganismen vorhanden sind.

Das Screening nach adhärenzdefekten H. pylori-Mutanten wird vorzugsweise zunächst mit Hilfe der KatoIII-Zellinie durchgeführt. Dazu können die Mutanten auf Agarplatten herangezogen und anschließend mit dem Fluoreszenz-Farbstoff Fluorescein-Isothiocyanat (FITC) markiert werden. Die markierten Bakterien werden dann vorzugsweise zu den Epithelzellen gegeben und 1 h bei 37 °C inkubiert. Anschließend wird mit Hilfe eines Fluoreszenzmikroskops überprüft, ob die einzelnen Mutanten noch zur Bindung an die Epithelzellinie in der Lage sind.

In einer bevorzugten Ausführungsform des erfindungsgemäßen Verfahrens legt man die H. pylori-Genbank in einem Minimal-Plasmidvektor an, der eine Selektion gegen eine Transposon-Insertion in die Vektor-DNA erlaubt. Bei Verwendung derartiger Minimal-Plasmidvektoren wird die Effizienz der Mutagene-Prozedur aufgrund der geringen Größe der genetischen Elemente des Plasmids stark verbessert. Weiterhin führt eine Insertion des Transposons in die Vektor-DNA, die im wesentlichen nur aus zur Replikation, Selektion und Klonierung notwendigen Elementen besteht, mit großer Wahrscheinlichkeit zu einem Verlust der Fähigkeit des Vektors, sich in der Wirtszelle zu propagieren.

Darüber hinaus ist es bevorzugt, wenn man die H. pylori-Genbank in einem Plasmidvektor anlegt, der eine zum konjugativen Transfer in andere Wirtsorganismen geeignete Sequenz umfaßt. Ein Beispiel für eine derartige Sequenz ist die oriT-Sequenz (Fürste et al., 1989), durch deren Anwesenheit die arbeitsintensive Isolierung von Plasmid-DNA nach der Induzierung des Transposons und der Transformation in geeignete Empfängerzellen vermieden werden kann.

Auch die Anwesenheit eines Expressionssignals auf dem Plasmidvektor ist bevorzugt, mit dem die insertierte H. pylori-DNA in operativer Verknüpfung steht. Auf diese Weise können Sequenzen von H. pylori-Genen transkribiert werden, die ansonsten aufgrund des Vorliegens spezialisierter Promotorsequenzen nicht transkribiert werden können. Außerdem können dann auch Gene transkribiert werden, die in Operons organisiert sind und die nicht als gesamte Einheit kloniert werden können. Die Verwendung eines schwachen Promotors ist bevorzugt, da stärkere Promotoren zu geringen Transformationsfrequenzen und sogar zu Deletionen und Umlagerungen der klonierten H. pylori-Sequenzen führen können.

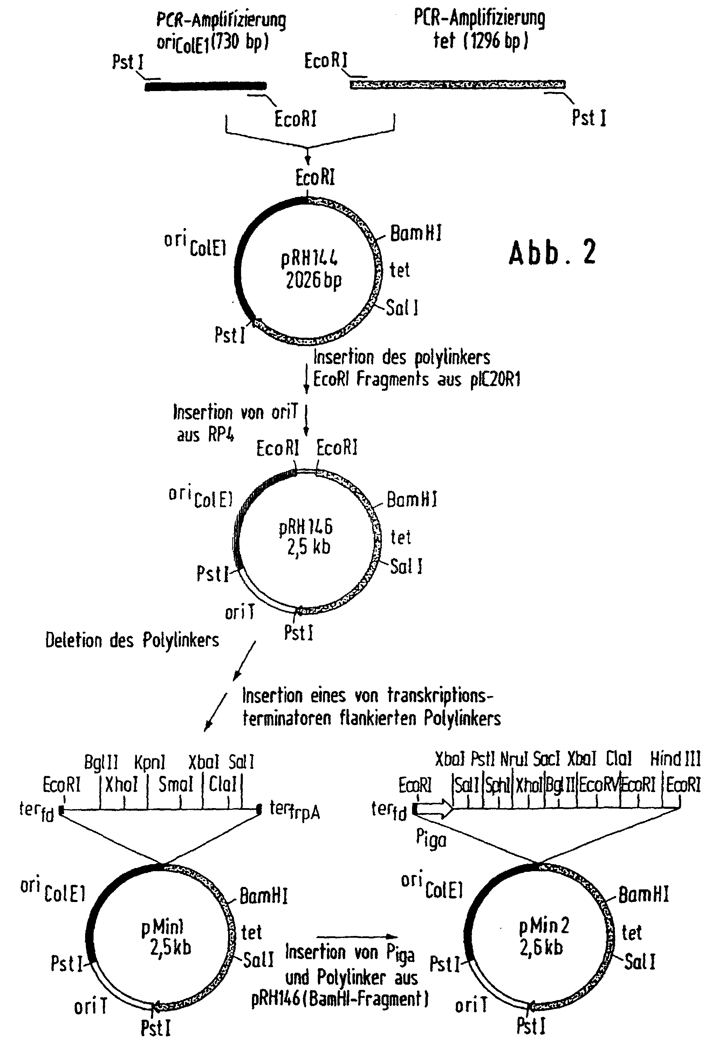

Ein besonders bevorzugter Plasmidvektor ist pMin2 (DSM 10007), der am 26.05.95 im E.coli Stamm DH5α gemäß den Vorschriften des Budapester Vertrags hinterlegt wurde. Die zur Konstruktion von pMin2 verwendeten Schritte sind schematisch in Abb. 2 dargestellt.

Wie bereits erwähnt, ist es für das erfindungsgemäße Verfahren bevorzugt, daß man für die Selektion auf Klone, die ein sekretorisches Gen enthalten, einen konjugativen Transfer von Plasmid-DNA aus dem Wirtsorganismus in einen Empfängerorganismus durchführt, der eine Selektion auf Transposon-enthaltende Plasmide erlaubt.

Vorzugsweise erfolgt die Selektion auf Adhärenzgene durch Retransformation von H. pylori, wobei durch Integration der DNA in das Chromosom ein Mutantenstamm erzeugt wird. Vor dieser Retransformation wird vorzugsweise das Markergen, z. B. das blaM-Gen deletiert, um eine höhere Transformationsfrequenz zu erhalten, die Erzeugung von H. pylori-Stämmen mit der Fähigkeit zur β-Lactamaseproduktion zu vermeiden und mögliche Störungen des bakteriellen Exportapparats durch BlaM-Fusionsproteine zu vermeiden.

Ein weiterer Gegenstand der vorliegenden Erfindung ist eine Genbank aus H. pylori-DNA in einem Wirtsorganismus, der H. pylori DNA-Fragmente inseriert in einem Vektor enthält, dadurch gekennzeichnet, daß das H. pylori-Genom mit einer Wahrscheinlichkeit von mehr als 90 % vollständig repräsentiert ist und daß der Wirtsorganismus weiterhin ein induzierbares Transposon gekoppelt mit einem Marker für sekretorische Aktivität enthält.

Bei einer Größe des H. pylori-Genoms von 1,7 Mb (Bukanov und Berg, 1994) und einer mittleren Größe der DNA-Insertion von vorzugsweise 3 - 6 kb und besonders bevorzugt von ca. 4 kb umfaßt die erfindungsgemäße Genbank vorzugsweise mindestens 400 Klone pro Genom. Besonders bevorzugt umfaßt die Genbank 2000 bis 4000 Klone.

Die H. pylori-DNA ist vorzugsweise in einem Plasmidvektor inseriert, besonders bevorzugt in einen Minimal-Plasmidvektor, wie oben definiert. Der Wirtsorganismus, in dem sich die Genbank befindet, ist vorzugsweise ein Bakterium, besonders bevorzugt ein gram-negatives Bakterium und am meisten bevorzugt E. coli.

Die erfindungsgemäße Genbank kann zur Identifizierung von sekretorischen Genen und insbesondere von Adhärenzgenen aus Helicobacter pylori verwendet werden.

Durch das erfindungsgemäße Verfahren konnte ein als alpA bezeichnetes Adhäsingen aus H. pylori und ein von diesem Gen codiertes Polypeptid identifiziert werden. Ein Gegenstand der vorliegenden Erfindung ist somit auch ein DNA-Molekül, das
(a) die in SEQ ID NO.1 dargestellte Nucleotidsequenz,
(b) eine der Sequenz gemäß (a) im Rahmen der Degeneration des genetischen Codes entsprechende Nucleotidsequenz oder
(c) eine mit den Sequenzen gemäß (a) oder/und (b) unter stringenten Bedingungen hybridisierende Nucleotidsequenz umfaßt.

Neben der in SEQ ID.1 gezeigten Nucleotidsequenz und einer dieser Sequenz im Rahmen der Degeneration des genetischen Codes entsprechenden Nucleotidsequenz umfaßt die vorliegende Erfindung auch noch eine DNA-Sequenz, die mit einer dieser Sequenzen unter stringenten Bedingungen hybridisiert. Der Begriff "Hybridisierung" gemäß vorliegender Erfindung wird wie bei Sambrook et al (Molecular Cloning. A Laboratory Manual, Cold Spring Harbor Laboratory Press (1989), 1.101 bis 1.104) verwendet. Gemäß vorliegender Erfindung spricht man von einer Hybridisierung unter stringenten Bedingungen, wenn nach Waschen für eine Stunde mit 1 X SSC und 0,1 % SDS bei 55 °C, vorzugsweise bei 62 °C und besonders bevorzugt bei 68 °C, insbesondere für eine Stunde in 0,2 X SSC und 0,1 % SDS bei 55 °C, vorzugsweise bei 62 °C und besonders bevorzugt bei 68 °C noch ein positives Hybridisierungssignal beobachtet wird. Eine unter derartigen Waschbedingungen mit einer der in SEQ ID NO.1 gezeigten Nucleotidsequenz oder einer damit im Rahmen der Degeneration des genetischen Codes entsprechenden Nucleotidsequenz hybridisierende Nucleotidsequenz wird von der vorliegenden Erfindung umfaßt.

Vorzugsweise codiert das erfindungsgemäße DNA-Molekül für ein Polypeptid mit der Fähigkeit zur Adhärenz an humane Zellen, insbesondere an humane Magenepithelzellen. Weiterhin ist bevorzugt, daß das erfindungsgemäße DNA-Molekül auf Nucleotidebene eine Homologie von mindestens 70 %, besonders bevorzugt von mindestens 80 % zu der in SEQ ID. NO.1 dargestellten Nucleotidsequenz aufweist. Weiterhin ist bevorzugt, daß das DNA-Molekül eine Länge von mindestens 15, besonders bevorzugt von mindestens 20 Nucleotiden aufweist.

Ein weiterer Gegenstand der vorliegenden Erfindung ist ein Vektor, der mindestens eine Kopie eines erfindungsgemäßen DNA-Moleküls enthält. Dieser Vektor kann ein beliebiger prokaryontischer oder eukaryontischer Vektor sein, auf dem sich die erfindungsgemäße DNA-Sequenz vorzugsweise unter Kontrolle eines Expressionssignals (Promotor, Operator, Enhancer etc.) befindet. Beispiele für prokaryontische Vektoren sind chromosomale Vektoren, wie etwa Bakteriophagen (z. B. Bakteriophage λ) und extrachromosomale Vektoren, wie etwa Plasmide, wobei zirkuläre Plasmidvektoren besonders bevorzugt sind. Geeignete prokaryontische Vektoren sind z. B. bei Sambrook et al., Supra, Kapitel 1 bis 4, beschrieben.

Andererseits kann der erfindungsgemäße Vektor auch ein eukaryontischer Vektor sein, z. B. ein Hefevektor oder ein für höhere Zellen geeigneter Vektor (z. B. ein Plasmidvektor, viraler Vektor, Pflanzenvektor). Derartige Vektoren sind beispielsweise bei Sambrook et al, Supra, Kapitel 16, beschrieben.

Ein weiterer Gegenstand der vorliegenden Erfindung ist eine Zelle, die mit einem erfindungsgemäßen Vektor transformiert ist. In einer bevorzugten Ausführungsform ist die Zelle eine prokaryontische Zelle, vorzugsweise eine gram-negative prokaryontische Zelle, besonders bevorzugt eine E. coli-Zelle. Andererseits kann die erfindungsgemäße Zelle jedoch auch eine eukaryontische Zelle sein, wie etwa eine Pilzzelle (z. B. Hefe), eine tierische oder eine pflanzliche Zelle.

Die Erfindung betrifft auch ein Polypeptid, welches von einem erfindungsgemäßen DNA-Molekül codiert ist. Vorzugsweise besitzt das Polypeptid die Fähigkeit zur Adhärenz an humane Zellen und umfaßt (a) die in SEQ. ID NO.2 dargestellte Aminosäuresequenz oder (b) eine mit der Sequenz gemäß (a) immunologisch kreuzreagierende Aminosäuresequenz.

Besonders bevorzugt weist das erfindungsgemäße Polypeptid eine Homologie von mindestens 80 % und am meisten bevorzugt von mindestens 90 % mit der in SEQ ID NO.2 dargestellten Aminosäuresequenz auf.

Die Herstellung von erfindungsgemäßen Polypeptiden erfolgt vorzugsweise dadurch, daß man eine Zelle mit einem erfindungsgemäßen DNA-Molekül oder Vektor transformiert, die transformierte Zelle unter Bedingungen kultiviert, bei denen eine Expression des Polypeptids stattfindet und das Polypeptid aus der Zelle oder/und aus dem Kulturüberstand isoliert. Dabei kann das erfindungsgemäße Polypeptid sowohl als Fusionspolypeptid als auch als Nicht-Fusionspolypeptid gewonnen werden.

Das erfindungsgemäße Polypeptid kann als Immunogen zur Herstellung von Antikörpern verwendet werden. Die vorliegende Erfindung betrifft somit auch einen Antikörper, der gegen ein erfindungsgemäßes Polypeptid gerichtet ist. Vorzugsweise ist der Antikörper gegen den N-Terminus, z. B. die ersten 250 Aminosäuren der in SEQ ID NO.2 dargestellten Aminosäuresequenz gerichtet.

Noch ein weiterer Aspekt der vorliegenden Erfindung betrifft eine pharmazeutische Zusammensetzung, die als Wirkstoff ein erfindungsgemäßes DNA-Molekül, ein erfindungsgemäßes Polypeptid oder einen erfindungsgemäßen Antikörper, gegebenenfalls zusammen mit üblichen pharmazeutischen Hilfs-, Verdünnungs-, Zusatz- und Trägermitteln enthält.

Die erfindungsgemäße pharmazeutische Zusammensetzung kann einerseits zur Diagnostik einer Helicobacter pylori-Infektion verwendet werden. Die Diagnostik auf Nucleinsäureebene erfolgt vorzugsweise durch Verwendung von Hybridisierungssonden, welche eine erfindungsgemäße, für das alpA-Gen spezifische DNA-Sequenz enthalten, oder durch Amplifikation unter Verwendung erfindungsgemäßer DNA-Moleküle als Primer. Auf Proteinebene erfolgt die Diagnostik vorzugsweise mit Hilfe der erfindungsgemäßen Antikörper.

Andererseits kann die pharmazeutische Zusammensetzung auch zur Prävention oder Bekämpfung einer Helicobacter pylori-Infektion verwendet werden. Vorzugsweise werden für therapeutische Anwendungen das Polypeptid oder Teile davon zur Herstellung eines aktiven Impfstoffs oder der Antikörper zur Herstellung eines passiven Impfstoffs verwendet.

Weiterhin soll die Erfindung durch die nachfolgenden Beispiele und Abbildungen erläutert werden.
- Abb. 1: zeigt die lineare Restriktionskarte des Transposons TnMax9 (DSM 10008), das zur Identifizierung und Inaktivierung des alpA-Gens benutzt wurde.
- Abb. 2: zeigt eine schematische Darstellung der Konstruktion der Minmal-Plasmidvektoren pMin1 und pMin2 (DSM 10007), die für eine effiziente Transposon-Mutagenese geeignet sind.
- Abb. 3A: zeigt das Selektionsprinzip der Transposon-Mutagenese mit TnMax9 und das Schema zur Erzeugung von H. pylori-Mutanten, die einen Defekt in einem sekretorischen Gen aufweisen.
- Abb. 3B: zeigt eine schematische Darstellung der Prozedur für die Identifizierung von H. pylori-Mutanten, die einen Defekt in einem sekretorischen Gen aufweisen.
- Abb. 4: zeigt eine Restriktionskarte des Plasmids pMu140, welches die regulatorische Region und das 5'-Ende des alpA-Gens (SEQ ID NO.1) enthält. Das alpA-Gen wird durch Insertion des Transposons TnMax9 inaktiviert (siehe Dreieck mit Beschriftung TnMax9). Bei Expression des Plasmids wird ein alpA-β-Lactamase-Fusionsprotein erhalten. pMu140 ist der ursprüngliche Klon aus der Mutanten-Genbank, aus dem durch Retransformation und homologe Rekombination der adhärenzdefekte H. pylori-Stamm P1-140 erhalten wurde.
- Abb. 5: zeigt einen Immunoblot von H. pylori-Gesamtzell-Lysaten des Wildtypstamms 69A (1) sowie den isogenen Mutantenstämmen P1-140 (2) und P1-179a (3). Der Wildtypstamm 69A enthält das alpA-Protein mit einem Molekulargewicht von ca. 53 kDa. Die alpA-Mutanten P1-140 und P1-179a weisen kein AlpA-Protein auf. Der Immunoblot wurde mit dem Antikörper AK 202 entwickelt, der gegen den N-Terminus des rekombinanten AlpA-Proteins gerichtet ist.
- SEQ ID NO.1: zeigt die Nucleotidsequenz des H. pylori-Adhärenzgens alpA und die entsprechende Aminosäuresequenz.
- SEQ ID NO.2: zeigt die Aminosäuresequenz des AlpA-Adhärenzpolypeptids aus H. pylori.

### Beispiel 1

### Konstruktion von Minimalvektoren

Das Plasmid pRH144 wurde durch Ligation des ColE1-Replikationsursprungs (ori_{ColE1}) und des Tetracyclin-Resistenzgens (Tet) hergestellt, die beide als PCR-Fragmente aus pBR322 unter Verwendung der Primerpaare RH104 (5'-AGC TGA ATT CAT GTT TGA CAT TGC CAT ATA GAT GAG CTT TAA TGC GGT AGT T-3') und RH105 (5'-AGC TCT GCA GCC GCC GGC TTC CAT TCA G-3') bzw. RH106 (5'-AGC TCT GCA GAG ATC AAA GGA TCT TCT T-3' und RH107 (5'-TCT AGA ATT CGT ATC AGC TCA CTC AAA G-3') amplifiziert wurden.

pRH146 wurde durch Insertion eines oriT-Fragments hergestellt, das aus dem Plasmid RP 4 (Marsh et al., 1984) durch PCR-Amplifizierung mit dem Primerpaar DF001 (5'-GTA CTG CAG CTT GGT TTC ATC AGC CA-3') und DF002 (5'-GTA CTG CAG TTC AGT AAT TTC CTG CAT-3') erhalten wurde.

Anschließend wurde die multiple Klonierungsstelle des Plasmids pIC20R1 (Thomas, 1981) in die EcoRI-Stelle von pRH146 insertiert. Um das Plasmid pMin1 zu erhalten, wurde der Polylinker von pRH146 durch eine doppelsträngige synthetische Polylinkerregion ersetzt, die in die EcoRI-Stelle von pRH146 kloniert wurde und aus den beiden, teilweise komplementären Oligonucleotiden RH117 (5'-AAT TAG ATC ATT AAA GGC TCC TTT TGG AGC CTT TTT TTT TGA ATT CAG ATC TCG AGG TAC CCG GGA TCC TCT AGA-3') und RH118 (5'-AAT TAG ATC AAA AAA AAA GCC CGC TCA TTA GGC GGG CTA AGC TTG TCG ACA TCG ATC TAG AGG ATC CCG GGT ACC-3') zusammengesetzt war.

Die flankierenden EcoRI-Stellen wurden durch die Klonierungsprozedur zerstört. Die klonierte Polylinkerregion enthielt die Transkriptionsterminatoren des Phagen fd (ter_{fd}) und des trpA-Gens (ter_{trpA}).

Um das Plasmid pMin2 zu erhalten, wurde der Promotor des aus Gonokokken stammenden iga-Gens (P_{iga}) als PCR-Fragment aus dem Plasmid pIP 100 (Pohlner et al., 1987) unter Verwendung der Oligonucleotide SO009 (5'-GGA TCC GAA TTC TCA TGT TTG ACA G-3') und SO010 (5'-GTC GAC AGA TCT TTT AAT AGC GAT AAT GT-3') amplifiziert. Das Amplifikationsfragment wurde in die EcoRI und BglII-Stellen von pRH160 ligiert. Der trpA-Terminator wurde durch Ersetzen des BglII/BamHI-Fragments von pMin1 durch das korrespondierende Fragment von pRH146 entfernt.

Das Konstruktionsschema für pMin1 und pMin2 ist in Abb. 2 dargestellt. Der das Plasmid pMin enthaltende E.coli Stamm DH5α wurde bei DSM unter dem Aktenzeichen 10007 hinterlegt.

### Beispiel 2

### Konstruktion des Transposons TnMax9

Als Basis für die Konstruktion von pTnMax9 wurde das Plasmid pRH110 benutzt, ein Derivat von pTnMax1 (Haas et al., 1993) mit einem SalI-linearisierten pIC20R2-Vektor in der singulären SalI-Schnittstelle. Anschließend wurde der zentrale Bereich von TnMax1 (ori_{fd}-res-cat_{GC}) durch HindIII-Spaltung und Religation deletiert. Die Sequenzen für die M13-forward (M13-Primer) und M13-reverse (M13-RP1)-Sequenzierprimer wurden durch Insertion der beiden teilweise komplementären Oligonucleotide RH096 (5'-AGC TTA CTG GCC GTC GTT TTA CAG CGG CCG CAG GAA ACA GCT ATG ACC GA-3') und RH097 (5'-AGC TTC GGT CAT AGC TGT TTC CTG CGG CCG CTG TAA AAC GAC GGC CAG TA-3') in die HindIII-Schnittstelle von pRH110 eingeführt (Ligation). Das resultierende Plasmid pRH140 enthielt eine singuläre NotI-Restriktionsschnittstelle zwischen den beiden Primerbindungsstellen. Das res DNA-Fragment von Tn1721 wurde mit Hilfe des Oligonucleotid-Primerpaares RH098 (5'-AGA AGC GGC CGC AAA AGG ATC CAT AGG TGC AAG CAA GTT A-3') und RH099 (5'-AGC TGC GGC CGC AAA AAG ATC TCA AAG CCC ATT TCT GTC AGG-3') vom Plasmid pJOE106 (Schöffl et al., 1981) amplifiziert und in die pRH140 NotI-Schnittstelle inseriert. Daraus entstand Plasmid pRH141, das singuläre BamHI und BglII-Restriktionsschnittstellen links und rechts von res aufwies. Nun wurde der Replikationsursprung (ori_{fd}) zusammen mit der cat_{GC}-Resistenz als BamHl-Fragment aus pRH42 isoliert und in die BamHl-Spaltstelle von pRH141 ligiert. Die Deletion des pIC20R2-Vektors (mit SalI) führte schließlich zu pTnMax5, dem Basis-Konstrukt für alle weiteren TnMax-Transposons. Schließlich wurde das β-Lactamase-Gen ohne Promotor und ohne Signalsequenz (blaM-Gen) vom Plasmid pBR322 (Sutcliffe, 1979) mit Hilfe der Oligonucleotide RH124 (5'-AGT TGC GGC CGC ACC CAG AAA CGC TGG TG-3') und RH127 (5'-AGC TAG ATC TAG ATT ATC AAA AAG GAT C-3') über PCR amplifiziert und in die NotI- und BglII-Spaltstellen von TnMax5 insertiert, woraus pTnMax7 resultierte. Die NotI-Schnittstelle zwischen cat_{GC} und dem inverted repeat (IR) von pTnMax7 wurde entfernt durch Auffüllen der überhängenden Enden mit Klenow-Polymerase. Die BglII-Schnittstelle am 3'-Ende von blaM wurde durch Insertion des Komplementären Oligonucleotid-Paares SO012 (5'-GAT CAA GTC GCG GCC GCC TGA T-3') und SO013 (5'-GAT CAT CAG GCG GCC GCG ACT T-3') in eine NotI-Schnittstelle überführt, was zum Transposon-Derivat pTnMax9 führte.

Der das Transposon-Derivat pTnMax9 enthaltende E.coli Stamm E 181 wurde bei DSM unter dem Aktenzeichen 10008 hinterlegt.

### Beispiel 3

### Herstellung einer H. pylori-Plasmid-Genbank

Es wurde eine Plasmidgenbank von der chromosomalen DNA des H. pylori-Wildtyp Stammes 69A angelegt. Dazu wurde die chromosomale DNA nach der Methode von Leying et al. ( 1992) aus H. pylori isoliert und mit den Restriktionsendonukleasen Sau3AI und HpaII jeweils partiell gespalten. Anschließend wurden die DNA-Fragmente auf einem präparativen Agarosegel aufgetrennt und Fragmente von 3 - 6 kb wurden aus dem Gel eluiert. Diese DNA-Fragmente wurden in den speziell dafür konstruierten Plasmidvektor pMin2, der mit den Restriktionsenzymen BglII und ClaI geschnitten war, ligiert (T4-Ligase) und der Ligationsansatz wurde in den E. coli-Stamm E181, ein den lysogenen λPhagen λCH616 enthaltendes Derivat des Stammes HB101 (Bayer und Roulland-Dussoix, 1969) transformiert, der bereits mit dem Transposon TnMax9 transformiert war. Dabei wurden ca. 2400 unabhängige Transformanden erhalten.

### Beispiel 4

### Isolierung von H. pylori-Mutanten

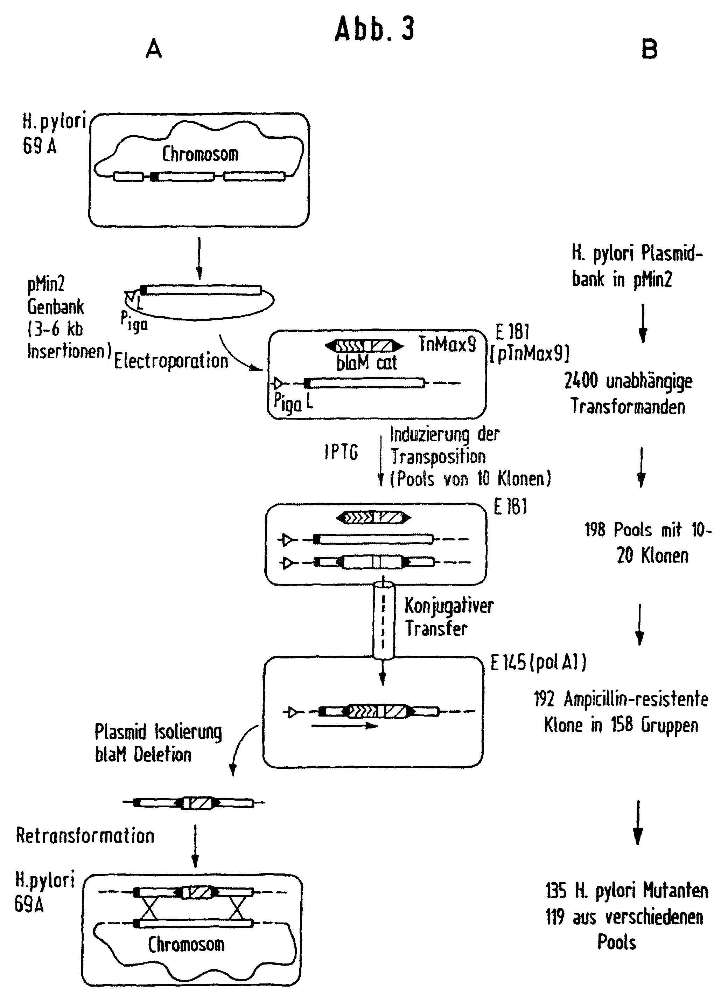

Das Selektionsprinzip der Mutagenese und die Prozedur für die Identifizierung von Mutanten sind schematisch in Abb. 3A und 3B dargestellt.

Zur Durchführung der Transposon-Mutagenese wurden jeweils 10 Transformanden vereinigt und gemeinsam induziert, wodurch insgesamt 190 Pools ä 10-20 Klonen weiterbehandelt wurden. Aus dieser Mutagenese wurden 191 Ampicillin-resistente E. coli- Plasmidklone isoliert, die unabhängige mutierte H. pylori-Gene trugen. Diese 192 Plasmide wurden aus E. coli isoliert und zur Retransformation des H. pylori-Stammes 69A benutzt. Aus diesen 192 Transformationen wurden 135 H. pylori-Mutanten isoliert, die in Genen mutiert sein sollten, die für sekretorische Proteine kodieren. Die H. pylori-Mutantenkollektion wurde dann in einem Screening-Assay auf H. pylori-Mutanten getestet, die ihre Fähigkeit an KatoIII Epithelzellen zu binden, verloren hatten.

Hierzu wurden die Mutanten mit FITC markiert und 1 h bei 37 °C gemeinsam mit den Epithelzellen kultiviert. Der Test auf Adhärenz erfolgte direkt durch Betrachtung mit einem Fluoreszenzmikroskop. Dabei wurden 2 Mutanten gefunden (Nr. P1-140 und P1-179a), die eine stark verminderte Adhärenz zeigten.

Beide Mutanten zeigten auch in dem zweiten Adhärenz-Modell, den Gewebeschnitten von menschlichen Magen, keine Adhärenz. Der H. pylori-Wildtyp-Stamm sowie alle weiteren Mutanten zeigten auch in diesem Modell eine starke Adhärenz.

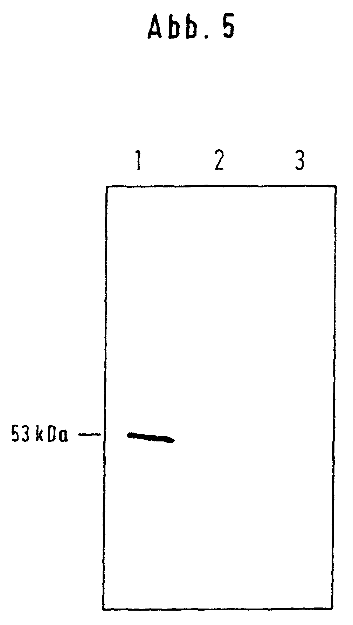

Das zur Erzeugung des Mutantenstammes P1-140 verwendete Plasmid pMu140 ist in Abb. 4 dargestellt. Zur Erzeugung des Mutantenstammes P1-179a wurde das Plasmid pMu179a (nicht gezeigt) verwendet. Unabhängige Transformationen beider Plasmide in H. pylori 69A führten zu dem identifizierten Adhärenzdefekt, was belegte, daß keine sekundären Mutationen im bakteriellen Chromosom aufgetreten waren, sondern die TnMax9 Insertion im klonierten Adhäsingen zu dem beobachteten Phänotyp der H. pylori-Mutanten führte. Die Kartierung und Sequenzierung der durch das Transposon TnMax9 inaktivierten Gene der Plasmidklone pMu140 und pMu179 zeigte, daß es sich bei beiden Klonen um dasselbe Gen handelte, das Transposon war nur an verschiedenen Stellen insertiert. Da es sich bei dem kodierten Protein um ein Lipoprotein handelt, also ein Protein, das mit einem Lipidanker in der Membran verankert ist, bezeichneten wir das entsprechende Gen als alpA, adherence- associated lipoprotein A). Unsere Daten aus Computer-Sekundärstrukturvorhersagen von Membranproteinen und von bestimmten konservierten Proteinsequenzen am C-Terminus des Proteins (C-terminales Phenylalanin) (Struyvé et al., 1991) sprechen für ein in die äußere Membran der gramnegativen Bakterien eingelagertes integrales Membranprotein.

### Beispiel 5

### Expression des alpA-Gens und Herstellung von Antikörpern

Mit Hilfe des pEV40 E. coli-Expressionssystems (Pohlner et al., 1993) wurde dann ein Fusionsprotein hergestellt, das ca 50 % vom N-Terminus von AlpA umfaßt und ein N-terminales Histidin-Tag aufweist. Damit wurde das Fusionsprotein mittels einer Ni2-Agarose-Säule durch Chelat-Affinitätschromatographie aufgereinigt und zur Gewinnung eines Antiserums von der Maus eingesetzt. Dieses Antiserum, welches die Bezeichnung AK202 erhielt, erkannte das AlpA Protein im Immunoblot eines H. pylori-Gesamtzellysates als 53 kDa Protein. Das 53 kDa AlpA Protein war bei den H. pylori-Mutanten P1-140 und P1-179a im Immunoblot erwartungsgenäß nicht detektierbar (siehe Abb. 5). Der Nachweis, daß es sich bei AlpA um ein Lipoprotein handelt, wurde durch die spezifische Palmityilierung des AlpA- β-Lactamase Fusionsproteins geführt, das durch die ursprüngliche Insertion von TnMax9 in alpA entstand.

### Literatur

Alper, J. (1993) Ulcers as an infectious disease. Science 260: 159-160.

Bizzozero, G. (1893) Ueber die schlauchförmigen Drusen des Magendarmkanals und die Beziehungen ihres Epithels zu dem Oberflächenepithel der Schleimhaut. Arch Mikr Anast 42: 82.

Blaser, M.J. (1992) Hypotheses on the pathogenesis and natural history of Helicobacter pylori induced inflammation. Gastroenterol 102: 720-727

Borén, T., Falk, P., Roth, K.A., Larson, G., Normark, S. (1993) Attachment of Helicobacter pylori to gastric epithelium mediated by blood group antigens. Science 262: 1892-1895.

Boyer, H.W. and Roulland-Dussoix, D. (1969) A complementation analysis of the restriction and modification of DNA in Escherichia coli. J Mol Biol. 41:459-472.

Bukanov, N.O., Berg, D.E. (1994) Ordered cosmid library and high-resolution physical-genetic map of Helicobaster pylori strain NCTC11638. Mol Microbiol 11: 509-523.

Clyne, M., Drumm, B. (1993) Adherence of Helicobacter pylori to primary human gastrointestinal cells. Infect Immun 61: 4051-4057.

Doig, P., Austin, J.W., Kostrzynska, M., Trust, T.J. (1992) Production of a conserved adhesin by the human gastroduodenal pathogen Helicobacter pylori. J Bacteriol 174: 2539-2547.

Doig, P., Austin, J.W., Trust, T.J. (1993) The Helicobacter pylori 19.6-kilodalton protein is an iron-containing protein resembling ferritin. J Bacteriol 175: 557-560.

Evans, D.G., Karjalainen, T.K., Evans, D.J., Jr., Graham, D.Y., Lee, C.-H. (1993) Cloning, nucleotide sequence, and expression of a gene encoding an adhesin subunit protein of Helicobacter pylori. J Bacteriol 175: 674-683.

Falk, P., Roth, K.A., Borén, T., Westblom, T.U., Gordon, J.I., Normark, S. (1993) An in vitro adherence assay reveals that Helicobacter pylori exhibits cell lineage-specific tropism in the human gastric epithelium. Proc Natl Acad Sci USA 90: 2035-2039.

Fürste, J.P., Pansegrau, W.,Ziegelin, G.,Kröger, M. and Lanka, E. (1989) Conjugative transfer of promiscuous IncP plasmids: interaction of plasmid-encoded products with the transfer origin. Proc Natl Acad Sci USA 86: 1771-1775.

Goodwin, C.S., Armstrong, J.A., Chilvers, T., Peters, M., Collins, M.D., Sly, L., McConnell, W., Harper, W.E.S. (1989) Transfer of Campylobacter pylori and Campylobacter mustelae to Helicobacter gen. nov. as Helicobacter pylori comb. nov. and Helicobacter mustelae comb. nov., respectively. Int J Syst Bact 39: 397-405.

Haas, R., Kahrs, A.F., Facius, D., Allmeier, H., Schmitt, R., Meyer, T.F. (1993) TnMax- a versatile mini-transposon for the analysis of cloned genes and shuttle mutagenesis. Gene 130: 23-31.

Isaacson, P.G., Spencer, J. (1993) Is gastric lymphoma an infectious disease? Hum Pathol 24: 569-570.

Lee, A., Fox, J., Hazell, S. (1993) Pathogenicity of Helicobacter pylori: a perspective. Infect Immun 61: 1601-1610.

Leying, H., Suerbaum, S., Geis, G., Haas, R. (1992) Characterisation of flaA, a Helicobacter pylori flagellin gene. Mol Microbiol 6: 2863-2874.

Mai, U.E.H., Perez-Perez, I., Wahl, L.M., Wahl, S.M., Blaser, M.J., Smith, P.D. (1991) Soluble surface proteins from Helicobacter pylori activate monocytes/macrophages by lipopolysaccharide-independent mechanism. J Clin Invest 87: 894-900.

Malfertheiner P. (1994) Helicobacter pylori - Von der Grundlage zur Therapie. Bayerdörfer E, Birkholz S, Börsch G. et al., (eds.) Stuttgart, New York: Georg Thieme Verlag; p. 1-104.

Marsh, J.L., Erfle, M. and Wykes, E.J. (1984) The pIC plasmid and phage vectors with versatile cloning sites for recombinant selection by insertional inactivation. Gene 32: 481-485.

Marshall, B.J., Royce, H., Annear, D.I., Goodwin, C.S., Pearman, J.W., Warren, J.R., Armstrong, J.A. (1984) Original isolation of Campylobacter pyloridis from human gastric mucosa. Microbios Lett 25: 83-88.

Michetti, P., Corthesy-Theulaz, I., Davin, C., Haas, R., Vaney, A.C., Heitz, M., Bille, J., Kraehenbuhl, J.P., Saraga, E., Blum, A.L. (1994) Immunization of Balb/c mice against Helicobacter felis infection with Helicobacter pylori urease. Gastroenterol 107: 1002-1011.

Nomura, A., Stemmermann, G.N., Chyou, P.H., Kato, I., Perez-Perez, G.I., Blaser, M.J. (1991) Helicobacter pylori infection and gastric carcinoma among japanese americans in Hawaii. N Engl J Med 325: 1132-1136.

Parkin, D.M., Läärä, E., Muir, C.S. (1988) Estimates of the worldwide frequency of sixteen maior cancers in 1980. Int J Cancer 41: 184-197.

Parsonnet, J., Friedman, G.D., Vandersteen, D.P., Chang, Y., Vogelman, J.H., Orentreich, N., Sibley, R.K. (1991) Helicobacter pylori infection and the risk of gastric carcinoma. N Engl J Med 325:1227-1131.

Pohlner, J., Krämer, J. and Meyer, T.F. (1993) A plasmid system for high-level expression and in vitro processing of recombinant proteins. Gene 130:121-126.

Pohlner, J. Halter, R., Beyreuther, K. and Meyer, T.F. (1987) Gene structure and extracellular secretion of Neisseria gonorrhoeae IgA protease. Nature 325:458-462.

Pugsley, A.P. (1993) The complete general secretory pathway in gram-negative bacteria. Microbiol Rev 57: 50-108.

Schöffl, F. Pühler, A., Altenbuchner, J. and Schmitt, R. (1981) The tetracycline resistance transposons Tn1721 and Tn 1771 have three 38-base-pair repeats and generate fivebase-pair repeats. Mol Gen Genet 181:87-94.

Sipponen, P. (1992) Natural history of gastritis and its relationship to peptic ulcer disease. Digestion 51: 70-75.

Sipponen, P., Hyvärinen, H. (1993) Role of Helicobacter pylori in the pathogenesis of gastritis, peptic ulcer and gastric cancer. Scand J Gastroenterol 28: 3-6.

Solnick, J.V., Tompkins, L.S. (1993) Helicobacter pylori and gastroduodenal disease: pathogenesis and host-parasite interaction. Infect Ag Dis 1: 294-309.

Stolte, M., Eidt, S. (1993) Healing gastric MALT lymphomas by eradicating H pylori. Lancet 342: 568.

Struyvé, M., Moons, M., Tommassen, J. (1991) Carboxyterminal phenylalanine is essential for the correct assembly of a bacterial outer membrane protein. J Mol Biol 218: 141- 148.

Sutcliffe, J.G. (1979) Complete nucleotide sequence of the Escherichia coli plasmid pBR322. Cold Spring Harb Symp Quant Biol 43:77-90.

Tadayyon, M., Broome-Smith, J.K. (1992) TnblaM - a transposon for directly tagging bacterial genes encoding cell envelope and secreted proteins. Gene 111: 21-26.

Taylor, D.N., Blaser, M.J. (1991) The epidemiology of Helicobacter pylori infection. Epidemiol Rev 13: 42-59.

Thomas C.M. (1981) Molecular genetics of broad host range plasmid RK2. Plasmid 5:10-19.

Warren, J.R., Marshall, B. (1983) Unidentified curved bacilli on gastric epithelium in active chronic gastritis. Lancet i: 1273- 1275.

## Patentansprüche

1. Verfahren zur Identifizierung sekretorischer Gene aus Helicobacter pylori,
**dadurch gekennzeichnet,**
**daß** man
(a) eine Genbank von H. pylori DNA in einem Minimal-Plasmidvektor, der im wesentlichen nur aus zur Replikation, Selektion und Klonierung notwendigen Elementen besteht und eine Selektion gegen eine Transposon-Insertionin die Vektor-DNA erlaubt, in einem Wirtsorganismus anlegt, der ein induzierbares Transposon gekoppelt mit einem Marker für sekretorische Aktivität enthält,
(b) die Insertion des Transposons in die H. pylori-DNA induziert und
(c) mit Hilfe des Markers eine Selektion auf Klone durchführt, die ein sekretorisches Gen enthalten.

2. Verfahren nach Anspruch 1 zur Identifizierung von Adhärenzgenen aus Helicobacter pylori,
**dadurch gekennzeichnet,**
**daß** man weiterhin
(d) eine Retransformation von H. pylori mit der DNA von Klonen, die Gene mit sekretorischer Aktivität enthalten, durchführt, wobei durch Integration der DNA in das Chromosom isogene H. pylori-Mutantenstämme erzeugt werden und
(e) eine Selektion auf Adhärenz-defiziente H. pylori-Mutantenstämme durchführt.

3. Verfahren nach einem der Ansprüche 1 bis 2,
**dadurch gekennzeichnet,**
**daß** man die H. pylori-Genbank in einem Plasmidvektor anlegt, der eine zum konjugativen Transfer in andere Wirtsorganismen geeignete Sequenz umfaßt.

4. Verfahren nach einem der Ansprüche 1 bis 3,
**dadurch gekennzeichnet,**
**daß** man die H. pylori-Genbank in einen Plasmidvektor anlegt, in dem die insertierte H. pylori-DNA in operativer Verknüpfung mit einem im Wirtsorganismus aktiven Expressionssignal steht.

5. Verfahren nach einem der Ansprüche 1 bis 4,
**dadurch gekennzeichnet,**
**daß** man als Plasmidvektor pMin2 (DSM 10007) verwendet.

6. Verfahren nach einem der Ansprüche 1 bis 5,
**dadurch gekennzeichnet,**
**daß** man als Marker für die sekretorische Aktivität ein β-Lactamasegen ohne Signalsequenz verwendet.

7. Verfahren nach einem der Ansprüche 1 bis 6,
**dadurch gekennzeichnet,**
**daß** man als Transposon TnMax9 (DSM 10008) verwendet.

8. Verfahren nach einem der Ansprüche 1 bis 7,
**dadurch gekennzeichnet,**
**daß** die Selektion auf Klone, die ein sekretorisches Gen enthalten, einen konjugativen Transfer von Plasmid-DNA aus dem Wirtsorganismus in einen Empfängerorganismus umfaßt, der eine Selektion auf Transposon enthaltende Plasmide erlaubt.

9. Genbank aus H. pylori-DNA in einem Wirtsorganismus, der H. pylori DNA-Fragmente inseriert in einen Vektor enthält,
**dadurch gekennzeichnet,**
**dass** der Vektor ein Minimal-Plasmidvektor ist, der im wesentlichen nur aus zur Replikation, Selektion und Klonierung notwendigen Elementen besteht und eine Selektion gegen eine Transposon-Insertion in die Vektor-DNA erlaubt, dass die Größe der Genbank derart ist, das H. pylori Genom mit einer Wahrscheinlichkeit von mehr als 90 % vollständig repräsentiert ist und dass der Wirtsorganismus weiterhin ein induzierbares Transponson gekoppelt mit einem Marker für sekretorische Aktivität enthält.

10. Genbank nach Anspruch 9,
**dadurch gekennzeichnet,**
**daß** der Wirtsorganismus ein Bakterium ist.

11. Genbank nach einem der Ansprüche 9 bis 10,
**dadurch gekennzeichnet,**
**daß** der Wirtsorganismus E. coli ist.

12. Genbank nach einem der Ansprüche 9 bis 11,
**dadurch gekennzeichnet,**
**daß** die H. pylori DNA-Fragmente eine mittlere Größe von 3 - 6 Kb aufweisen.

13. Genbank nach einem der Ansprüche 9 bis 12,
**dadurch gekennzeichnet,**
**daß** sie mindestens 400 unabhängige Klone umfaßt.

14. Verwendung einer Genbank nach einem der Ansprüche 11 bis 13 zur Identifizierung sekretorischer Gene aus Helicobacter pylori.

15. Verwendung nach Anspruch 14 zur Identifizierung von Adhärenzgenen aus Helicobacter pylori.

## Claims

1. Method for identifying secretory genes from Helicobacter pylori,
wherein
(a) a gene bank of H. pylori DNA in a minimal plasmid vector which comprises essentially only elements necessary for replication, selection and cloning and allows selection against a transposon insertion into the vector DNA is set up in a host organism which contains an inducible transposon coupled with a marker for secretory activity,
(b) the insertion of the transposon into the H. pylori DNA is induced and
(c) the marker is used to select for clones which contain a secretory gene.

2. Method as claimed in claim 1 for identifying adherence genes from Helicobacter pylori,
wherein
in addition
(d) H. pylori is retransformed with the DNA of clones which contain genes with secretory activity, which produces isogenic H. pylori mutant strains by integration of the DNA into the chromosome and
(e) adherence-deficient H. pylori mutant strains are selected.

3. Method as claimed in one of the claims 1 to 2,
wherein
the H. pylori gene bank is set up in a plasmid vector which contains a sequence suitable for conjugative transfer into other host organisms.

4. Method as claimed in one of the claims 1 to 3,
wherein
the H. pylori gene bank is set up in a plasmid vector in which the inserted H. pylori DNA is in operative linkage with an expression signal that is active in the host organism.

5. Method as claimed in one of the claims 1 to 4,
wherein
pMin2 (DSM 10007) is used as the plasmid vector.

6. Method as claimed in one of the claims 1 to 5,
wherein
a β-lactamase gene without a signal sequence is used as a marker for the secretory activity.

7. Method as claimed in one of the claims 1 to 6,
wherein
TmMax9 (DSM 10008) is used as the transposon.

8. Method as claimed in one of the claims 1 to 7,
wherein
the selection for clones which contain a secretory gene comprises a conjugative transfer of plasmid DNA from the host organism into a recipient organism that allows selection for plasmids containing a transposon.

9. Gene bank of H. pylori DNA in a host organism which contains H. pylori DNA fragments inserted into a vector,
wherein
the vector is a minimal plasmid vector which comprises essentially only elements necessary for replication, selection and cloning and allows selection against a transposon insertion into the vector DNA, the size of the gene bank is such that the H. pylori genome is completely represented with a probability of more than 90 % and the host organism additionally contains an inducible transposon coupled with a marker for secretory activity.

10. Gene bank as claimed in claim 9,
wherein
the host organism is a bacterium.

11. Gene bank as claimed in one of the claims 9 to 10,
wherein
the host organism is E. coli.

12. Gene bank as claimed in one of the claims 9 to 11,
wherein
the H. pylori DNA fragments have an average size of 3- 6 kb.

13. Gene bank as claimed in one of the claims 9 to 12,
wherein
it comprises at least 400 independent clones.

14. Use of a gene bank as claimed in one of the claims 11 to 13 for identifying secretory genes from Helicobacter pylori.

15. Use as claimed in claim 14 for identifying adherence genes from Helicobacter pylori.

## Revendications

1. Procédé d'identification de gènes sécrétoires obtenus à partir de *Helicobacter pylori,* **caractérisé en ce que** :
(a) on dispose une banque de gènes de l'ADN de *H. pylori* dans un vecteur plasmide minimal, qui n'est constitué essentiellement que par les éléments nécessaires à la réplication, la sélection et le clonage et qui permet une sélection par rapport à une insertion de transposon dans l'ADN de vecteur, dans un organisme hôte, qui contient un transposon inductible couplé à un marqueur d'activité sécrétoire,
(b) on induit l'insertion du transposon dans l'ADN de *H. pylori*, et
(c) on réalise une sélection à l'aide du marqueur, des clones qui contiennent un gène sécrétoire.

2. Procédé selon la revendication 1, pour l'identification de gènes d'adhérence obtenus à partir d'*Helicobacter pylori,* **caractérisé en ce qu'**en plus :
(d) on réalise une retransformation de *H. pylori* avec l'ADN des clones, qui contiennent le gène avec activité sécrétoire, où par intégration de l'ADN dans le chromosome, on produit des souches mutantes isogènes de *H. pylori,* et
(e) on réalise une sélection de souches mutantes de *H. pylori* déficientes au niveau de l'adhérence.

3. Procédé selon l'une quelconque des revendications 1 et 2, **caractérisé en ce que** l'on dispose la banque de gène de *H. pylori* dans un vecteur plasmide, qui comprend une séquence appropriée pour le transfert par conjugaison dans d'autres organismes hôtes.

4. Procédé selon l'une des revendications 1 à 3, **caractérisé en ce que** l'on dispose la banque de gène de *H. pylori* dans un vecteur plasmide, dans lequel l'ADN inséré de *H. pylori* se trouve en liaison fonctionnelle avec un signal d'expression actif dans l'organisme hôte.

5. Procédé selon l'une quelconque des revendications 1 à 4, **caractérisé en ce que** l'on utilise comme vecteur plasmide, pMin2 (DSM 10007).

6. Procédé selon l'une des revendications 1 à 5, **caractérisé en ce que** l'on utilise comme marqueur pour l'activité sécrétoire, un gène de S-lactamase sans séquence signal.

7. Procédé selon l'une des revendications 1 à 6, **caractérisé en ce que** l'on utilise comme transposon, TnMax9 (DSM 10008).

8. Procédé selon l'une des revendications 1 à 7, **caractérisé en ce que** la sélection des clones qui contiennent un gène sécrétoire, comprend un transfert par conjugaison d'ADN de plasmide depuis l'organisme hôte dans un organisme récepteur, qui permet une sélection de plasmides contenant le transposon.

9. Banque de gènes de l'ADN de *H. pylori* dans un organisme hôte, qui contient des fragments d'ADN de *H. pylori* insérés dans un vecteur, **caractérisée en ce que** le vecteur est un vecteur plasmide minimal, qui ne consiste essentiellement qu'en les éléments nécessaires à la réplication, la sélection et le clonage et permet une sélection par rapport à une insertion de transposon dans l'ADN de vecteur, et que la taille de la banque de gènes est telle que le génome de *H. pylori* est complètement représenté avec une probabilité de plus de 90% et que l'organisme hôte contient de plus, un transposon inductible couplé à un marqueur de l'activité sécrétoire.

10. Banque de gènes selon la revendication 9, **caractérisée en ce que** l'organisme hôte est une bactérie.

11. Banque de gènes selon l'une des revendications 9 et 10, **caractérisée en ce que** l'organisme hôte est *E. coli*.

12. Banque de gènes selon l'une des revendications 9 à 11, **caractérisée en ce que** les fragments d'ADN de *H. pylori* présentent une taille moyenne de 3 à 6 kb.

13. Banque de gènes selon l'une des revendications 9 à 12, **caractérisée en ce qu'**elle comprend au moins 400 clones indépendants.

14. Utilisation d'une banque de gène selon l'une des revendications 11 à 13, pour l'identification de gènes sécrétoires obtenus à partir de *Helicobacter pylori*.

15. Utilisation selon la revendication 14, pour l'identification de gènes d'adhérence obtenus à partir de *Helicobacter pylori*.
